(19) Europäisches Patentamt
European Patent Office
Office européen des brevets

(11) Numéro de publication: **0 225 823 B1**

(12) **FASCICULE DE BREVET EUROPEEN**

(45) Date de publication du fascicule du brevet: **31.01.90**

(51) Int. Cl.⁴: **C07D 475/08**

(21) Numéro de dépôt: **86402544.0**

(22) Date de dépôt: **17.11.86**

(54) **Procédé de préparation de dérivés de la ptéridine.**

(30) Priorité: **19.11.85 FR 8517057**

(43) Date de publication de la demande: **16.06.87 Bulletin 87/25**

(45) Mention de la délivrance du brevet: **31.01.90 Bulletin 90/5**

(84) Etats contractants désignés: **AT BE CH DE FR GB IT LI LU NL SE**

(56) Documents cités: **EP-A- 0 048 002**
**FR-A- 2 351 103**

(73) Titulaire: **RHONE-POULENC SANTE, 20, avenue Raymond Aron, F-92160 Antony(FR)**

(72) Inventeur: **Zimmermann, Pierre, Rue des Fontaines Les Buissonnières, F-69360 Chaponnay(FR)**

(74) Mandataire: **Pilard, Jacques et al, RHONE-POULENC SANTE Service Brevets Santé 25 Quai Paul Doumer, F-92408 Courbevoie Cédex(FR)**

EP 0 225 823 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dérivés de la ptéridine de formule générale :

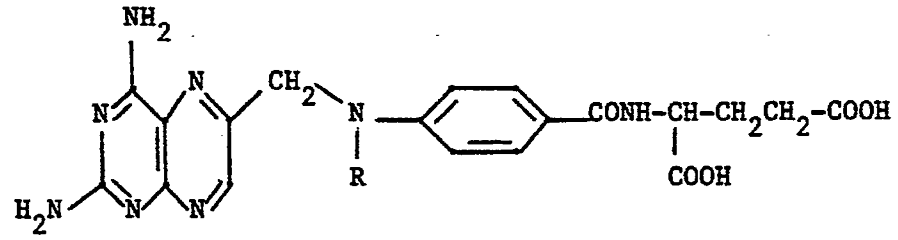

(I)

dans laquelle R représente un atome d'hydrogène ou le radical méthyle par amination d'un dérivé de la ptéridine de formule générale :

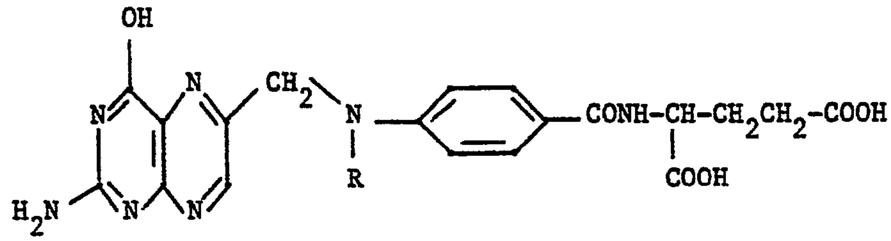

(II)

dans laquelle R est défini comme précédemment.

Le produit de formule générale (I) dans laquelle R represente un atome d'hydrogène est l'aminoptérine (acide N-[{N-[(diamino-2,4-ptéridinyl-6) méthyl] amino}-4 benzoyl] L-(+) glutamique).

Le produit de formule générale (I) dans laquelle R représente le radical méthyle est le méthotrexate (acide N-[{N-[(diamino-2,4 ptéridinyl-6) méthyl] N-méthylamino}-4 benzoyl] L-(+) glutamique) qui est un antimitotique utilisé comme antagoniste de l'acide folique dans le traitement des leucémies.

Le produit de formule générale (II) dans laquelle R représente un atome d'hydrogène est l'acide folique.

Le produit de formule générale (II) dans laquelle R représente le radical méthyle est le méthoptérine (acide N-[{N-[(amino-2 hydroxy-4 ptéridinyl-6) méthyl] N-méthylamino}-4 benzoyl] L-(+) glutamique).

Il est connu de préparer le méthotrexate selon le procédé décrit par D.R. Seeger et coll., J. Amer. Chem. Soc., 71, 1753 (1949) qui consiste à faire réagir, en une seule opération, le sulfate de tétramino-2,4,5,6 pyrimidine, le N-méthyl-p.aminobenzoylglutamate de sodium et le dibromopropionaldéhyde, cependant il n'existe pas de procédé permettant de transformer l'acide folique en méthotrexate.

Bien qu'il soit connu de préparer la méthoptérine par méthylation de l'acide folique au moyen de formol en présence de cyanoborohydrure de sodium à un pH voisin de 6,4 selon le procédé décrit par CAROLL TEMPLE JR et J.A. MONTGOMERY, J. Med. Chem., 25, 161 (1982), l'amination de la méthoptérine n'a pas été décrite.

D'après les brevets français FR 2 163 672 et FR 2 351 103, il est connu de préparer des dérivés aminés de composés hétérocycliques azotés par silylation d'hétérocycles substitués par un ou plusieurs radicaux hydroxy suivi de l'action de l'ammoniac ou d'une amine primaire ou secondaire en présence d'acide de Lewis ou d'acide p.toluènesulfonique. Comme agents de silylation peuvent être utilisés l'hexaméthyldisilazane éventuellement en présence de triméthylchlorosilane, l'hexaméthylcyclotrisilazane ou l'octaméthylcyclotétrasilazane. Lorsque l'on utilise l'ammoniac comme agent d'amination, il est nécessaire d'opérer sous une pression comprises entre 30 et 50 atmosphères, la température de la réaction atteignant 180°C.

Par ailleurs, il est connu d'après le brevet américain US 3 884 957 de préparer un nitrile par chauffage d'un acide carboxylique avec un silazane cyclique ou linéaire en présence d'un acide de Lewis tel que le chlorure d'aluminium ou le chlorure de zinc.

Il a maintenant été trouvé de manière surprenante et inattendue, et c'est ce qui fait l'objet de la présente invention, que l'amination de l'acide folique ou de la méthoptérine peut être réalisée dans des conditions qui permettent de remplacer un radical hydroxy par un radical amino sans toucher au reste de la molécule.

Plus particulièrement, il a été trouvé que l'acide folique ou la méthoptérine traités par un silazane conduisent respectivement à l'aminoptérine ou au méthotrexate avec de bons rendements sans qu'il soit nécessaire d'utiliser de l'ammoniac et d'opérer sous pression.

Généralement, le procédé d'amination selon l'invention est mis en oeuvre en chauffant l'acide folique ou la méthoptérine avec un silazane tel de l'hexaméthyldisilazane dans un solvant organique basique ou

en présence d'un catalyseur à caractère acide, dans un solvant organique basique ou l'acétonitrile.

Comme solvant organique, on utilise généralement la pyridine, la N,N-diméthylaniline ou la quinoléine. Il est particulièrement avantageux d'utiliser la pyridine.

Lorsque le procédé est mis en oeuvre en présence d'un catalyseur à caractère acide, il est particulièrement avantageux d'utiliser comme catalyseur un acide minéral ou organique tel que les acides chlorhydrique, sulfurique, formique, p.toluènesulfonique ou un sel à caractère acide obtenu à partir d'un acide et d'une base plus faible tel que les sels d'ammonium minéraux ou organiques comme les chlorure, sulfate ou formiate d'ammonium, le chlorhydrate de pyridinium, le p.toluènesulfonate de pyridinium ou le trifluoroacétate de N-méthyl-anilinium. Il est également possible d'utiliser un acide de Lewis tel que le chlorure de zinc comme catalyseur à caractère acide. Dans ces conditions, le procédé est mis en oeuvre dans un solvant organique basique ou dans l'acétonitrile.

D'un intérêt tout particulier est le p.toluènesulfonate de pyridinium dans l'acétonitrile.

Le silazane est généralement utilisé à raison de 2 à 10 moles par mole d'acide folique ou de méthoptérine mis en oeuvre.

Lorsque le procédé est mis en oeuvre en présence d'acide p.toluènesulfonique, on utilise, en générale, 0,01 à 0,2 mole de catalyseur par mole d'acide folique ou de méthoptérine mis en oeuvre.

La température de la réaction est comprise entre 60 et 180°C et la réaction est complète après 15 à 25 heures de chauffage à cette température.

L'aminoptérine ou le méthotrexate peuvent être séparés du mélange réactionnel selon les techniques habituelles d'extraction et ils peuvent être avantageusement isolés sous forme d'un sel métallique tel que le sel de sodium.

Le méthotrexate qui peut contenir de la méthoptérine non transformée peut être purifié par traitement dans les conditions de la réaction d'amination par l'hexaméthyldisilazane en présence de p.toluénesulfonate de pyridinium dans un solvant organique convenable tel que l'acétonitrile ou la pyridine.

Ce procédé peut être appliqué à la purification du méthotrexate issu de tous les procédés de fabrication conduisant au méthotrexate contaminé par de la méthoptérine.

Lorsque le procédé est mis en oeuvre à partir d'acide folique, l'aminoptérine obtenue peut être transformée en méthotrexate en opérant dans les conditions décrites par CAROLL TEMPLE JR et J.A. MONTGOMERY, J. Med. Chem., 25, 161 (1982) pour la préparation de la méthoptérine à partir de l'acide folique.

Les exemples suivants, donnés à titre non limitatif, montrent comment l'invention peut être mise en pratique.

## EXEMPLE 1

Dans un autoclave en hastelloy de 10 $cm^3$, on introduit 360 mg d'hydrate de méthoptérine (0,75 mmole), 3,6 $cm^3$ de pyridine anhydre, 21 mg d'acide p.toluènesulfonique monohydraté (0,11 mmole) et 1 $cm^3$ d'hexaméthyldisilazane (4,74 mmoles). On chauffe à 100°C sous agitation pendant 21,5 heures. Après refroidissement à 20°C, la solution pyridinique est concentrée à sec. Le résidu obtenu est repris par 10 $cm^3$ d'eau et on acidifie à pH = 3,3 par addition d'acide chlorhydrique normal. Le précipité jaune formé est séparé par filtration, rincé à l'eau et lavé avec 3 $cm^3$ d'éthanol. Après séchage sous pression réduite (0,2 torr) pendant 15 heures à 20°C, on obtient 340 mg de méthotrexate brut.

L'analyse par chromatographie liquide à haute performance montre que le taux de transformation est de 100 % et que le rendement est de 51,4 %.

On reprend 230 mg du produit obtenu précédemment par 5 $cm^3$ d'eau distillée. Le pH est ajusté à 9 par addition de soude normale. On sépare un léger insoluble par filtration. Au filtrat, on ajoute sous agitation 32 $cm^3$ d'acétone. Il se forme un précipité jaune qui est séparé par filtration et rincé à l'acétone. Après séchage sous pression réduite (0,5 torr) pendant 24 heures à 20°C, on obtient 140 mg d'un solide jaune dont le titre en sel de sodium du méthotrexate, déterminé par chromatographie liquide à haute performance, est de 81,8 %.

Le rendement de la purification est de 88 %.

La structure du produit obtenu est confirmée par le spectre infra-rouge, le spectre ultra-violet et le spectre de résonance magnétique nucléaire du proton.

## EXEMPLE 2

Dans un autoclave de 10 $cm^3$, on introduit 360 mg d'hydrate de méthoptérine (0,75 mmole), 3,6 $cm^3$ de pyridine anhydre et 1 $cm^3$ d'hexaméthyldisilazane (4,75 mmoles). On agite à 100°C pendant 21 heures 30 minutes.

Après refroidissement à une température voisine de 20°C, le mélange réactionnel est concentré à sec. Le résidu obtenu est repris par 10 $cm^3$ d'eau puis on acidifie à pH = 3,3 par addition d'acide chlorhydrique N. Le précipité jaune formé est séparé par filtration, rincé à l'eau puis lavé avec 3 $cm^3$ d'éthanol. Après séchage à 20°C sous pression réduite (1 mm de mercure ; 0,13 kPa), on obtient 300 mg de méthotrexate.

L'analyse par chromatographie liquide à haute performance montre que le taux de transformation de la méthoptérine est de 88 % et que le rendement de la réaction est de 48 %.

EXEMPLE 3

Dans un autoclave de 10 $cm^3$, on introduit 240 mg d'acide folique dihydraté (0,50 mmole), 5,48 $cm^3$ de pyridine anhydre, 0,65 $cm^3$ d'hexaméthyldisilazane (3,10 mmoles) et 17 mg d'acide p.toluènesulfonique monohydraté (0,083 mmole). On agite pendant 17 heures à 100°C. Après refroidissement à 20°C, le mélange réactionnel est concentré à sec. Le résidu obtenu est repris par 10 $cm^3$ d'eau puis on alcalinise à pH = 9 par addition de soude N. Un léger insoluble est séparé par filtration. Après addition de 80 $cm^3$ d'acétone, un solide jaune précipite qui est séparé par filtration et lavé par 5 $cm^3$ d'acétone. Après séchage à 20°C sous pression réduite (3 mm de mercure ; 0,04 kPa) pendant 15 heures, on obtient 240 mg de sel de sodium d'aminoptérine titrant 79 %.

Le taux de transformation de l'acide folique est de 99,4 % et le rendement est de 78,3 %.

La structure du produit obtenu est confirmée par le spectre de masse et le spectre de résonance magnétique nucléaire.

EXEMPLE 4

Dans un autoclave de 10 $cm^3$, on introduit 360 mg d'acide folique dihydraté (0,75 mmole), 3,6 $cm^3$ de pyridine anhydre et 1 $cm^3$ d'hexaméthyldisilazane (4,74 mmoles). On agite pendant 48 heures à 100°C. Après refroidissement à 20°C, le mélange réactionnel est concentré à sec. Le résidu obtenu est repris par 10 $cm^3$ d'eau puis on alcalinise à pH = 9 par addition de soude N. On ajoute alors 170 mg de chlorure de calcium dihydraté et 60 $cm^3$ d'acétone.

Le précipité formé est séparé par filtration, lavé avec 5 $cm^3$ d'acétone puis séché sous pression réduite (3 mm de mercure ; 0,4 kPa) pendant 15 heures à 20°C. On obtient ainsi 440 mg de sel de calcium d'aminoptérine titrant 33,3 %.

Le taux de transformation de l'acide folique est de 61,3 % et le rendement est de 41,3 %.

EXEMPLE 5

On met 400 mg de sel de calcium d'aminoptérine (0,84 mmole) en suspension dans 20 $cm^3$ d'eau distillée. On ajuste le pH à 6,4 par addition d'acide chlorhydrique N. On ajoute 0,34 $cm^3$ de formol à 37 % dans l'eau (4,20 mmoles) et 80 mg de cyanoborohydrure de sodium (1,27 mmole). On maintient le pH à 6,5 pendant 4 heures à 20°C par addition d'acide chlorhydrique N. Après 22 heures à 20°C, on amène le pH à 9 par addition de soude N. Un insoluble est séparé par filtration. Au filtrat, on ajoute 190 mg de chlorure de calcium dihydraté (1,26 mmole) puis 100 $cm^3$ d'acétone. Le précipité formé est séparé par filtration, rincé par 5 $cm^3$ d'acétone puis séché à 20°C pendant 15 heures sous pression réduite (3 mm de mercure ; 0,4 kPa).

On obtient ainsi 460 mg de sel de calcium du méthotrexate dont le titre est de 34,8 %.

EXEMPLE 6

Dans un autoclave de 250 $cm^3$, on introduit successivement 90 $cm^3$ d'acétonitrile, 9 g de méthoptérine titrant 96,8 %, 0,77 g d'acide paratoluènesulfonique monohydraté, 0,32 $cm^3$ de pyridine et 26,4 $cm^3$ d'hexaméthyldisilazane. On chauffe l'autoclave fermé à 100°C pendant 18 heures. La pression est de 2 bars. Après refroidissement à une température voisine de 20°C, le mélange réactionnel est concentré jusqu'au tiers de son volume (environ 40 $cm^3$). On ajoute 78 $cm^3$ d'eau distillée. Il se forme un précipité jaune. On élimine 60 $cm^3$ de solvant par distillation azéotropique sous pression réduite (60 mm de mercure ; 8 kPa). Le pH est égal à 4,8. On ajoute 130 $cm^3$ d'eau distillée puis ajuste le pH à 4 par addition de quelques gouttes d'acide chlorhydrique N. La suspension jaune est agitée pendant 1 heure à 20°C. Le précipité est séparé par filtration et il est lavé par 2 fois 100 $cm^3$ d'eau distillée.

Le précipité est repris par 90 $cm^3$ d'eau distillée et le pH est amené à 9 par addition de 33 $cm^3$ de soude N. A la solution obtenue on ajoute 384 $cm^3$ d'acétone. Un faible précipité se forme qui est séparé par filtration. Au filtrat, on ajoute 576 $cm^3$ d'acétone. Le sel de sodium du méthotrexate précipite. Après 1 heure à une température voisine de 20°C, le précipité est séparé par filtration. On lave le précipité par 3 fois 15 $cm^3$ d'acétone.

Le précipité est séché pendant 15 heures à 20°C sous pression réduite (0,1 mm de mercure ; 0,013 kPa).

On obtient ainsi 8,10 g de sel disodique de méthotrexate dont les caractéristiques sont les suivantes :
- titre (chromatographie liquide à haute performance) : 89,1 %
- eau : 7,8 %

Le taux de transformation de la méthoptérine est de 99,8 % et le rendement est de 75,7 %.

On dissout 8,10 g du sel de sodium de méthotrexate dans 47 $cm^3$ d'eau distillée. On chauffe la solution à 75°C puis on ajoute lentement 249 $cm^3$ d'éthanol jusqu'à l'obtention d'un trouble persistant. Après filtration à chaud, on laisse le filtrat revenir à une température voisine de 20°C en 1 heure environ puis on refroidit dans un bain de glace fondante pendant 2 heures 30 minutes. Le précipité jaune est séparé par filtration.

Le précipité est solubilisé dans 60 cm³ d'eau distillée puis on acidifie par addition d'acide chlorhydrique N jusqu'à pH = 4. Le précipité est séparé par filtration puis il est rincé par 20 cm³ d'eau distillée. Après séchage à 20°C, pendant 32 heures, sous pression réduite (0,1 mm de mercure ; 0,013 kPa), on obtient 6,57 g de méthotrexate, forme acide, titrant 95 %, la teneur en eau étant de 8,4 %.

Le rendement est de 95 %.

EXEMPLE 7

On introduit successivement dans un autoclave 3,6 cm³ de pyridine anhydre, 360 mg de méthotrexate sous forme acide, dont l'analyse par chromatographie liquide à haute performance montre qu'il contient 57,6 % de méthotrexate et 20,2 % de méthoptérine (pourcentange de surface), 22 mg d'acide paratoluènesulfonique, 1 cm³ d'hexaméthyldisilazane. On chauffe à 100°C pendant 16 heures. Après refroidissement, on évapore à sec. Le résidu est repris avec environ 10 cm³ d'eau distillée et amené à pH = 9 avec de la soude. On ajoute 170 mg de chlorure de calcium dihydraté. L'addition de 60 cm³ d'acétone conduit à la formation d'un précipité qui est séparé par filtration, essoré et rincé à l'acétone. Après séchage à 20°C pendant 15 heures sous pression réduite (3 mm de mercure ; 0,4 kPa), on obtient 440 mg de sel de calcium de méthotrexate, dont l'analyse par chromatographie liquide à haute performance montre qu'il contient 78,6 % de méthotrexate et 0,4 % de méthoptérine (pourcentage de surface).

EXEMPLE 8

On introduit dans un autoclave successivement 3,6 cm³ d'acétonitrile anhydre, 360 mg de méthotrexate sous forme de chlorhydrate, dont l'analyse par chromatographie liquide à haute performance montre qu'il contient 89,7 % de méthotrexate et 2,9 % de méthoptérine (pourcentage de surface), 25 mg d'acide paratoluènesulfonique, 10 cm³ de pyridine, 0,74 cm³ d'hexaméthyldisilizane. On chauffe à 100°C pendant 7 heures. Après refroidissement, l'analyse du mélange réactionnel par chromatographie liquide à haute performance montre la présence de 87,2 % de méthotrexate et l'absence de méthoptérine (pourcentage de surface).

## Revendications

1. Procédé de préparation de dérivés de la ptéridine de formule générale:

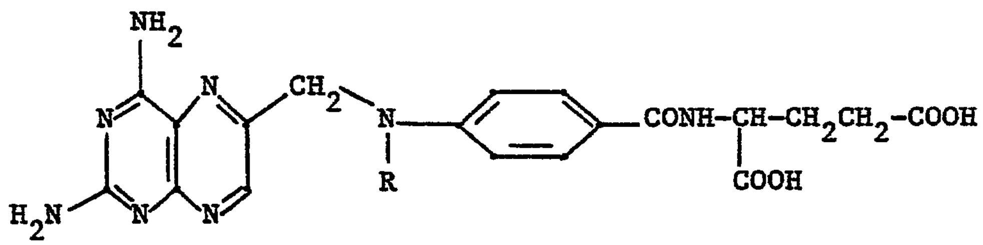

(I)

dans laquelle R représente un atome d'hyrogène ou le radical méthyle caractérisé en ce que l'on fait réagir un silazane sur un dérivé de la ptéridine de formule générale:

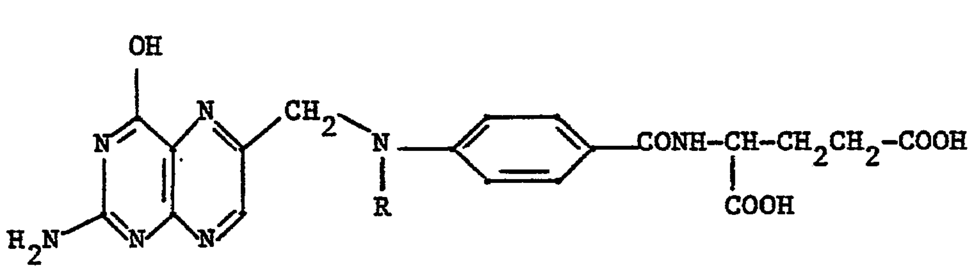

(II)

dans laquelle R est défini comme précédemment, en opérant à une température comprise entre 60 et 180°C, dans un solvant organique basique ou, en présence d'un catalyseur à caractère acide, dans un solvant organique basique ou dans l'acétonitrile et isole le produit obtenu éventuellement sous forme de sel métallique.

2. Procédé selon la revendication 1 caractérisé en ce que le silazane est l'hexaméthyldisilazane.

3. Procédé selon la revendication 1 caractérisé en ce que le solvant organique basique est choisi parmi la pyridine, la N,N-diméthylaniline et la quinoléine.

4. Procédé selon la revendication 1 caractérisé en ce que le catalyseur est choisi parmi les acides minéraux ou organiques, les sels d'ammonium minéraux ou organiques obtenus à partir d'un acide et d'une base plus faible et les acides de Lewis.

5. Procédé selon la revendication 4 caractérisé en ce que le catalyseur est choisi parmi les chlorure, sulfate ou formiate d'ammonium, le chlorhydrate ou le p.toluènesulfonate de pyridinium ou le trifluoroacétate de N-méthyl-anilinium.

6. Procédé selon la revendication 4 caractérisé en ce que le catalyseur est le p.toluènesulfonate de pyridinium.

7. Procédé selon la revendication 1 caractérisé en ce que l'on utilise le p.toluènesulfonate de pyrdinium dans l'acétonitrile.

8. Procédé selon l'une des revendications 1 à 7 caractérisé en ce que l'on utilise 2 à 10 moles de silazane par mole de dérivé de la ptéridine de formule générale (II) mis en œuvre.

9. Procédé selon la revendication 8 pour la préparation d'un dérivé de la ptéridine de formule générale (I) dans laquelle R représente le radical méthyle caractérisé en ce que l'on méthyle, au moyen de formol en présence de cyanoborohydrure de sodium, un dérivé de la ptéridine de formule générale (I) dans laquelle R représente un atome d'hydrogène obtenu par action d'un silazane sur un produit de formule générale (II) dans laquelle R représente un atome d'hydrogène.

10. Procédé pour la purification du méthotrexate contenant de la méthoptérine caractérisé en ce que l'on soumet le méthotrexate impur à l'action d'un silazane tel que l'hexaméthyldisilazane dans la pyridine en présence de p.toluènesulfonate de pyridinium à une température comprise entre 60 et 180°C et isole le méthotrexate purifié.

**Patentansprüche**

1. Verfahren zur Herstellung von Pteridinderivaten der allgemeinen Formel:

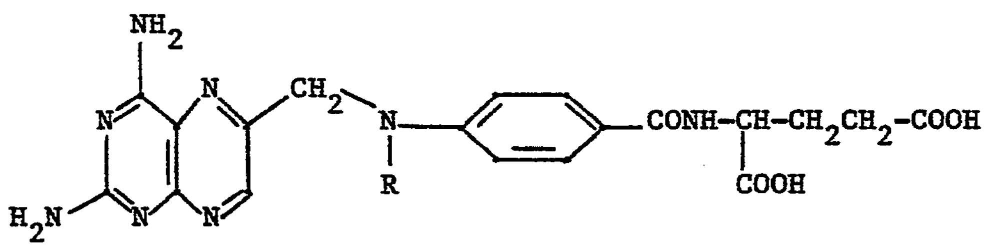

(I)

in welcher R ein Wasserstoffatom oder den Methylrest darstellt, dadurch gekennzeichnet, daß man ein Silazan mit einem Pteridinderivat der allgemeinen Formel:

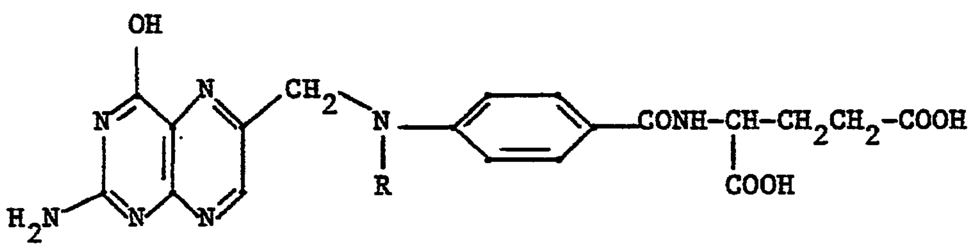

(II)

in welcher R die obige Bedeutung hat, umsetzt, wobei man bei einer Temperatur zwischen 60 und 180 °C in einem basischen organischen Lösungsmittel oder bei Anwesenheit eines Katalysators mit saurem Charakter in einem basischen organischen Lösungsmittel oder in Acetonitril arbeitet und die erhaltene Verbindung gegebenenfalls in Form des Metallsalzes isoliert.

2. Verfahren nach Anspruch 1, dadurch gekennzeichnet, daß das Silazan Hexamethyldisilazan ist.

3. Verfahren nach Anspruch 1, dadurch gekennzeichnet, daß das basische organische Lösungsmittel ausgewählt ist aus Pyridin, N,N-Dimethylanilin und Chinolin.

4. Verfahren nach Anspruch 1, dadurch gekennzeichnet, daß der Katalysator ausgewählt ist aus den anorganischen oder organischen Säuren, den aus einer Säure und einer schwächeren Base erhaltenen anorganischen oder organischen Ammoniumsalzen und den Lewis-Säuren.

5. Verfahren nach Anspruch 4, dadurch gekennzeichnet, daß der Katalysator ausgewählt ist aus Ammoniumchlorid, -sulfat oder -formiat, Pyridinchlorhydrat oder -p-toluolsulfonat oder N-Methylanilintrifluoracetat.

6. Verfahren nach Anspruch 4, dadurch gekennzeichnet, daß der Katalysator Pyridin-p-toluolsulfonat ist.

7. Verfahren nach Anspruch 1, dadurch gekennzeichnet, daß man Pyridin-p-toluolsulfonat in Acetonitril verwendet.

8. Verfahren nach einem der Ansprüche 1 bis 7, dadurch gekennzeichnet, daß man 2 bis 10 Mol Silazan pro Mol eingesetztem Pteridinderivat der allgemeinen Formel (II) verwendet.

9. Verfahren nach Anspruch 8 zur Herstellung eines Pteridinderivats der allgemeinen Formel (I), in welcher R den Methylrest darstellt, dadurch gekennzeichnet, daß man ein Pteridinderivat der allgemeinen Formel (I), in welcher R ein Wasserstoffatom darstellt und das erhalten wurde durch Umsetzung eines Silazans mit einer Verbindung der allgemeinen Formel (II), in welcher R ein Wasserstoffatom darstellt, mittels Formol in Gegenwart von Natriumcyanoborhydrid methyliert.

10. Verfahren zur Reinigung von Methotrexat, das Methopterin enthält, dadurch gekennzeichnet, daß man das unreine Methotrexat der Einwirkung eines Silazans, z.B. Hexamethyldisilazan, in Pyridin in Gegenwart von Pyridin-p-toluolsulfonat bei einer Temperatur zwischen 60 und 180°C unterwirft und das gereinigte Methotrexat isoliert.

**Claims**

1. A process for the preparation of pteridine derivatives of general formula:

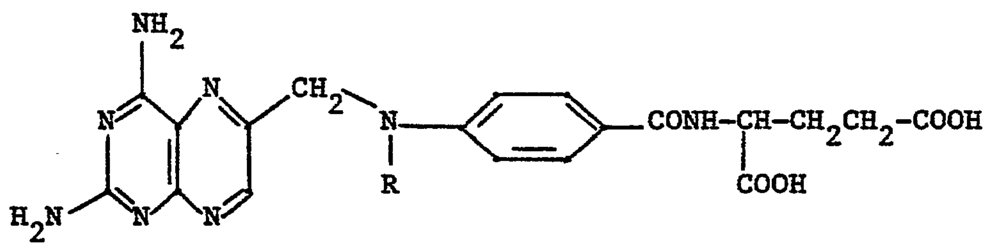

(I)

in which R represents a hydrogen atom or a methyl radical, characterised in that a silazane is reacted with a pteridine derivative of general formula:

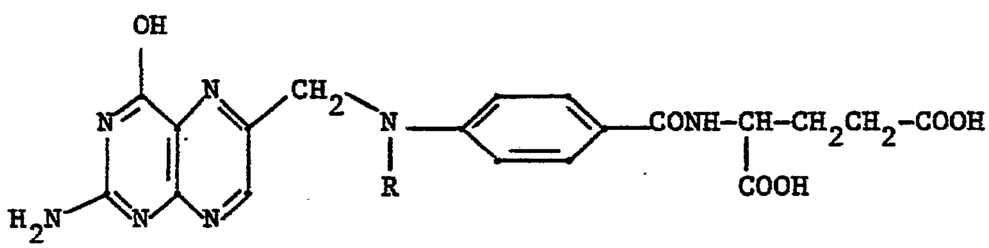

(II)

in which R is defined as above, by operating at a temperature between 60 and 180°C in a basic organic solvent or, in the presence of a catalyst which is acid in nature, in a basic organic solvent or in acetonitrile and the product obtained is isolated, if required, in the form of a metal salt.

2. The process according to claim 1, characterised in that the silazane is hexamethyldisilazane.

3. The process according to claim 1, characterised in that the basic organic solvent is chosen from pyridine, N,N-dimethylaniline and quinoline.

4. The process according to claim 1, characterised in that the catalyst is chosen from inorganic or organic acids, inorganic or organic ammonium salts obtained from an acid and a weaker base and Lewis acids.

5. The process according to claim 4, characterised in that the catalyst is chosen from ammonium chloride, sulphate or formate, pyridinium hydrochloride or p-toluenesulphonate or N-methylanilinium trifluoroacetate.

6. The process according to claim 4, characterised in that the catalyst is pyridinium p-toluenesulphonate.

7. The process according to claim 1, characterised in that the pyridinium p-toluenesulphonat is used in acetonitrile.

8. The process according to one of claims 1 to 7, characterised in that 2 to 10 moles of silazane are used per mole of pteridine derivative of general formula (II) employed.

9. A process according to claim 8 for the preparation of a pteridine derivative of general formula (I) in which R represents a methyl radical, characterised in that a pteridine derivative of general formula (I) in which R represents a hydrogen atom, obtained by the action of a silazane on a product of general formula (II) in which R represents a hydrogen atom, is methylated using formaldehyde in the presence of sodium cyanoborohydride.

10. A process for the purification of methotrexate containing methopterin, characterised in that the impure methotrexate is subjected to the action of a silazane such as hexamethyldisilazane in pyridine in the presence of pyridinium p-toluenesulphonate at a temperature between 60 and 180°C and the purified methotrexate is isolated.